# EUROPEAN PATENT APPLICATION

(11) **EP 4 417 967 A1**
(43) Date of publication of application: **21.08.2024**
(21) Application number: 22881182.4
(22) Date of filing: 08.08.2022
(51) Int. Cl.: G01N 23/046, G01N 23/18, G06N 3/08

(54) **CT SCAN APPARATUS**

(30) Priority: 15.10.2021 KR 20210137320
(71) Applicant: Pemtron Co., Ltd., Seoul 08501 (KR)
(72) Inventor: YOO, Young-Woong, Seoul 06574 (KR); CHO, Cheol-Hoon, Goyang-si, Gyeonggi-do 10333 (KR); YUN, Jeong-Sam, Gwangmyeong-si, Gyeonggi-do 14217 (KR); KIM, Yong- Ha, Seoul 06293 (KR)
(74) Representative: SJW Patentanwälte
(86) International application number: PCT/KR2022/011789
(87) International publication number: WO 2023/063551

(57) **Abstract**

The present invention relates to a CT scan apparatus, and comprises: a CT scan unit that captures N projection images of a subject to be examined; and a CT image generation unit having an artificial intelligence-based AI model that receives, as an input, the N projection images captured by the CT scan unit, and outputs a CT tomography image for the subject to be examined. Accordingly, a CT tomography image having high picture quality and resolution can be generated by capturing only a small number of projection images, for example, four projection images.

## Description

### Technical Field

The present disclosure relates to a CT scan apparatus and, more particularly, to a CT scan apparatus that is capable of generating a more accurate CT image with a small number of projection images.

### Background Art

In general, small electronic parts, such as a ball grid array (BGA) or a chip scale package (CSP), are mounted by soldering on a printed circuit board (PCB) mounted as a major component of electrical and electronic products such as home appliances and computers.

Therefore, in a process in which such a printed circuit board is mounted in a set of electrical and electronic products, the process of inspecting of whether the soldering condition of the printed circuit board mounted in an electronic part is good or bad is performed. In order to inspect the soldering condition of the printed circuit board, an X-ray inspection device has been mainly used recently.

An X-ray inspection device for such a printed circuit board is supplied inside a cabinet manufactured to define an indoor space for shielding to irradiate a printed circuit board set at an inspection location with X-rays, and determines whether the soldering condition of the soldered part of the printed circuit board is good or bad through image information output by capturing a projected image with a detector.

A conventional X-ray inspection device applies a tomographic image algorithm of laminography that obtains a cross-sectional layer for one image by synchronously rotating an X-ray generator and a detector with a subject to be examined placed therebetween.

However, the steerable X-ray generator applied to this conventional X-ray inspection device is complicated in configuration and high in manufacturing costs, and the detector with a large area is also expensive component, which is a factor in increasing the unit cost of the X-ray inspection device.

Another conventional inspection device using X-rays applies a tomographic image algorithm of tomosynthesis that obtains a cross-sectional image by numerically combining multiple X-ray images while rotating an X-ray generator.

The x-ray generator of tomosynthesis which is non-steerable is less expensive but has fewer functions than the x-ray generator of laminography which is steerable. However, This X-ray generator has a wide beam irradiation angle, that is, the beam irradiation angle of at least 100°, and is a component more expensive than a general X-ray generator.

In order to solve the above problems, a low-cost computer tomography (CT) scan device was developed that partially scans a subject to be examined, reconstructs image data acquired through the scanning into a tomography image, and is capable of determining defects through the 3D tomography image.

A conventional CT scan apparatus captures multiple images, that is, projection images, of an electronic part that is a subject to be examined from multiple directions, and reconstructs the images into one CT image, and the resolution and image quality of the finally generated CT image are determined by the number of the projection images.

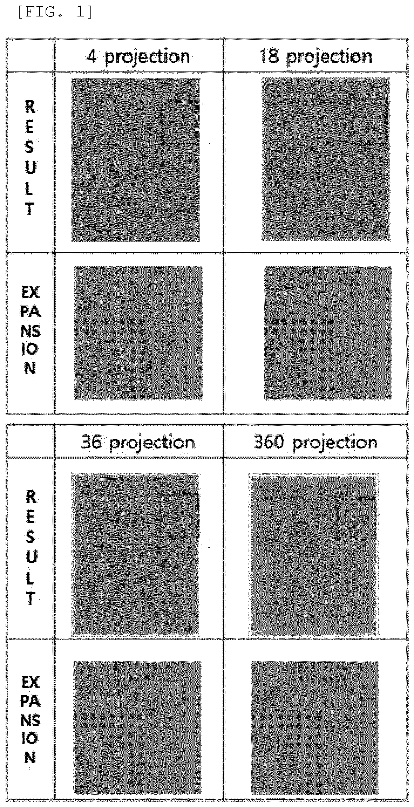

FIG. 1 is a view illustrating examples of CT tomography images generated according to the number of projection images in a conventional CT scan apparatus CT. As illustrated in FIG. 1, it is possible to check that as the number of the projection images increases, the image quality and resolution of CT tomography images increase.

In general, a CT tomography image generated by using at least 8 projection images is recognized as a minimum value for determining whether the quality of a part is good or bad, but it is known that it is sometimes impossible to determine whether some parts are good or bad even in the case of a CT tomography image generated through 8 projection images.

However, unlike medical images, it is realistically impossible to capture a large amount of projection images of each electronic part by a CT scan apparatus for examining an electronic part in order to improve resolution or image quality.

That is, if a lot of time is spent to examine whether the quality of an electronic part is good or bad in the in-line process, it causes a delay in an entire manufacturing process, which not only causes problems with production volume but also acts as a factor in increasing the price of a product.

Therefore, technology for generating CT tomography images with image quality and resolution sufficient to enable the determination of whether the quality of a part is good or bad even with a small amount of projection images is recognized as an important element in actual production lines.

In addition, with regard to a CT scan apparatus, it would be preferable if a CT scan apparatus for medical or dental use could generate a CT tomography image with high image quality and high-resolution by capturing a small number of projection images, thereby reducing examination time.

### [Document of Related Art]

### [Patent Document]

(Patent Document 1) Korean Patent No. 10-1717678

### Disclosure

### Technical Problem

The present disclosure has been made keeping in mind the above problems occurring in the prior art, and is intended to propose a CT scan apparatus which is capable of generating a more accurate CT tomography image with a small number of projection images.

### Technical Solution

In order to accomplish the above objectives, a CT scan apparatus according to the present disclosure includes: a CT scan unit configured to capture N projection images of a subject to be examined, and a CT image generation unit having an artificial intelligence-based AI model configured to receive the N projection images captured by the CT scan unit and output a CT tomography image of the subject to be examined.

Here, the AI model may be generated by learning N learning projection images, a first learning CT tomography image generated by the N learning projection images, and a second learning CT tomography image generated by M learning projection images (here, M > N) as learning data, wherein the AI model may learn the second learning CT tomography image as output data.

In addition, the AI model may learn the N learning projection images and the first learning CT tomography image as input data; and the CT image generation unit may further include a preprocessing module configured to process the N projection images to generate a CT tomography image for input and input the CT tomography image to the AI model.

In addition, N may be 4, and M may be 8 or more.

### Advantageous Effects

According to the present disclosure, provided is the CT scan apparatus which is capable of generating a CT tomography image having high picture quality and resolution by capturing only a small number of projection images, for example, four projection images.

In addition, due to the capturing of the small number of projection images, examination time is shortened, thereby enabling real-time CT scanning in an in-line process.

### Description of Drawings

FIG. 1 is a view illustrating examples of CT tomography images generated according to the number of projection images in a conventional CT scan apparatus CT,
FIG. 2 is a view illustrating the configuration of a CT scan apparatus according to an embodiment of the present disclosure,
FIG. 3 is a view illustrating an example of the capturing of a CT scan unit of the CT scan apparatus according to an embodiment of the present disclosure,
FIG. 4 is views illustrating examples of learning data applied to an AI model of the CT scan apparatus according to an embodiment of the present disclosure, and
FIGS. 5 and 6 are views illustrating the results of CT tomography images generated by the CT scan apparatus according to an embodiment of the present disclosure.

### Best Mode

The present disclosure relates to a CT scan apparatus, and includes a CT scan unit that captures N projection images of a subject to be examined, and a CT image generation unit having an artificial intelligence-based AI model that receives, as an input, the N projection images captured by the CT scan unit and outputs a CT tomography image for the subject to be examined.

### Mode for Invention

The advantages and features of the present disclosure, and how to achieve them will be clear by referring to embodiments described in detail below along with the accompanying drawings. However, the present disclosure is not limited to the embodiments disclosed below, but may be implemented in various forms. These embodiments are provided solely to ensure that the disclosure of the present disclosure is complete and to fully inform those skilled in the art to which the present disclosure belongs of the scope of the present disclosure. The present disclosure is only defined by the scope of the claims.

Terms used herein are intended to describe embodiments and are not intended to limit the present disclosure. In this specification, singular forms also include plural forms, unless specifically stated otherwise in the context. As used in the specification, "comprises" and/or "comprising" does not exclude the presence or addition of one or more other elements in addition to mentioned elements. Like reference numerals refer to like elements throughout the specification, and "and/or" includes each and every combination of one or more of the referenced elements. Although "first", "second", etc. are used to describe various components, these components are of course not limited by these terms. These terms are merely used to distinguish one component from another. Accordingly, a first component mentioned below may also be a second component within the technical idea of the present disclosure.

Unless otherwise defined, all terms (including technical and scientific terms) used in this specification may be used to have meanings commonly understood by those skilled in the art to which the present disclosure pertains. Additionally, terms defined in commonly used dictionaries are not interpreted ideally or excessively unless clearly and specifically defined.

Hereinafter, the present disclosure will be described in detail with reference to the accompanying drawings. In addition, in an embodiment of the present disclosure, a CT scan apparatus 100 is illustrated to be applied to the examination of an electronic part.

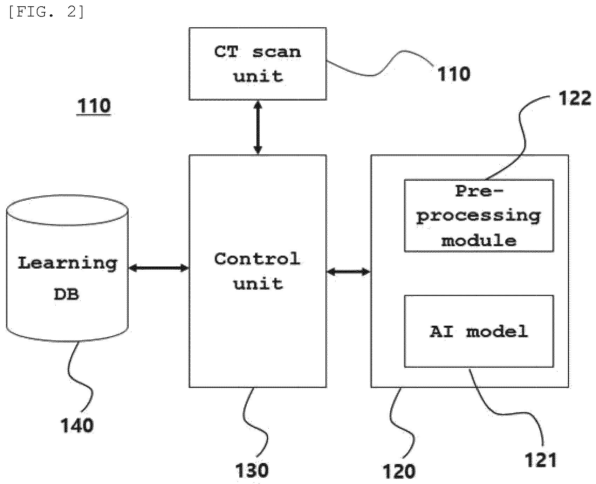

FIG. 2 is a view illustrating the configuration of the CT scan apparatus 100 according to the embodiment of the present disclosure.

Referring to FIG. 2, the CT scan apparatus 100 according to the embodiment of the present disclosure may include a CT scan unit 110 and a CT image generation unit 120. In addition, the CT scan apparatus 100 according to the embodiment of the present disclosure may further include a control unit 130.

The CT scan unit 110 according to the embodiment of the present disclosure takes N projection images of the electronic part. In the embodiment of the present disclosure, as an example, the CT scan unit 110 takes four projection images. When compared to M learning projection images used for training an AI model 121 to be described later, N is a natural number smaller than M, and a detailed description thereof will be provided later.

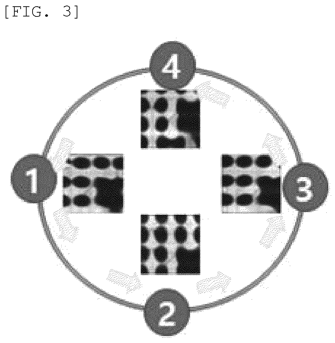

In an embodiment of the present disclosure, as an example, the CT scan unit 110 captures four projection images at 90° intervals as illustrated in FIG. 3, but capturing direction thereof is not limited thereto.

Meanwhile, the CT image generation unit 120 receives the N projection images captured by the CT scan unit 110, for example, the four projection images as described above, and generates the CT tomography image of the electronic part.

In the embodiment of the present disclosure, as an example, the CT image generation unit 120 includes the artificial intelligence-based AI model 121, and as an example, the artificial intelligence-based AI model 121 receives N projection images and infers and outputs a high-quality CT tomography image.

Through this, the artificial intelligence-based AI model 121 is able to use a pre-trained learning model to generate a CT tomography image having relatively high image quality and resolution with only the four projection images.

Meanwhile, as an example, the AI model 121 according to the embodiment of the present disclosure is generated by learning N learning projection images, a first learning CT tomography image generated by the N learning projection images, and a second learning CT tomography image generated by the M learning projection images as learning data. Here, one piece of the first learning CT tomography image and one piece of the second learning CT tomography image are learned.

Here, the AI model 121 learns the second learning CT tomography image as output data, and as described above,M is a natural number greater than N.

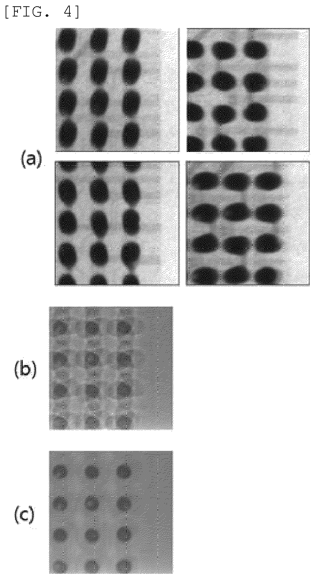

FIG. 4 is views illustrating examples of learning data applied to the training of the AI model 121 of the CT scan apparatus 100 according to an embodiment of the present disclosure. Here, the learning data may be stored in a learning data base 140.

As illustrated in FIG. 4(a), four learning projection images taken of a specific electronic part are input as a set of learning data. In addition, the first learning CT tomography image illustrated in FIG. 4(b) is a CT tomography image generated by using the four learning projection images illustrated in FIG. 4(a).

In addition, the second learning CT tomography image illustrated in FIG. 4(c) is a CT tomography image generated by using M projection images taken of the corresponding electronic part. In the embodiment of the present disclosure, as an example, the second learning CT tomography image used for learning is generated by using 8 or more projection images. In order to increase the learning performance of the AI model 121, as an example, a CT tomography image generated by using 360 projection images is applied as the second learning CT tomography image.

The AI model 121 learns by using the learning data described above, wherein the learning is performed by using the four learning projection images and the first learning CT tomography image as input data and by using the second learning CT tomography image as output data.

Here, the CT image generation unit 120 according to the embodiment of the present disclosure may include a preprocessing module 122 that processes the N projection images taken by the CT scan unit 110 to generate one CT tomography image for input and inputs the CT tomography image as input data to the AI model 121.

That is, the control unit 130 controls the preprocessing module 122 to generate a CT tomography image for input by using the N projection images captured by the CT scan unit 110, and the AI model 121 receives the N projection images captured by the CT scan unit 110 and the CT tomography image for input generated by the preprocessing module 122, and infers and outputs a CT tomography image.

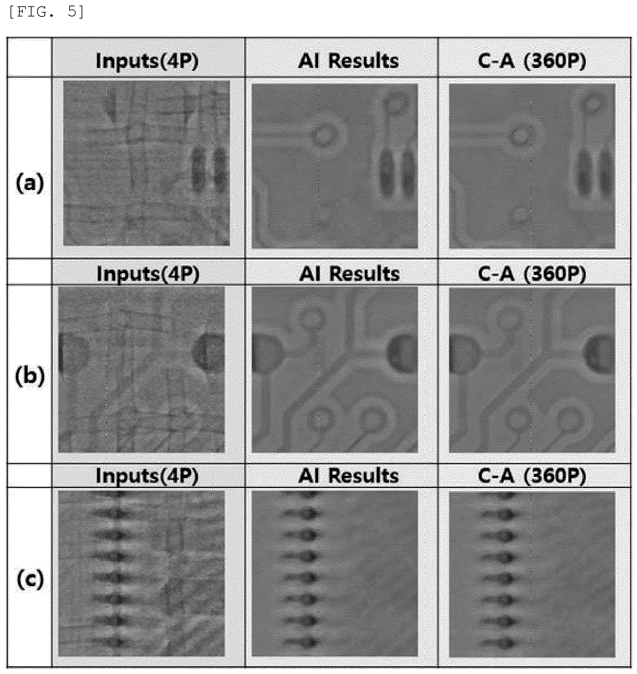

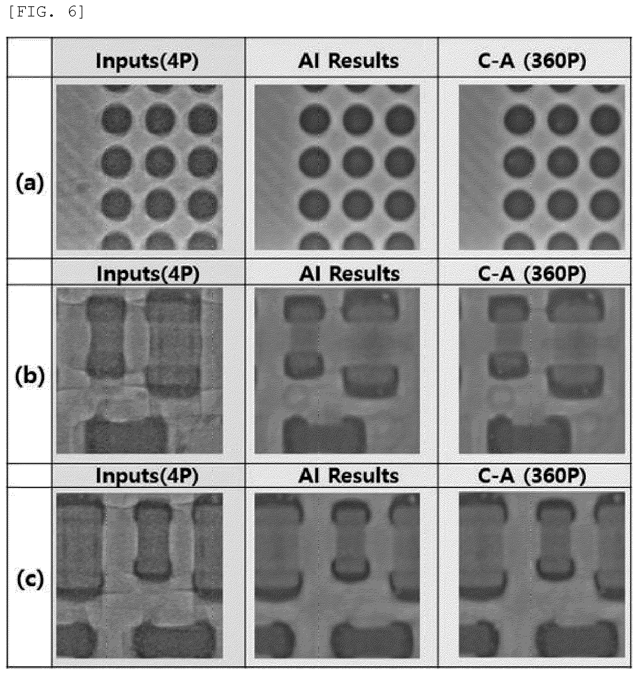

FIGS. 5 and 6 are views illustrating the examples of CT tomography images output by the CT scan apparatus 100 according to the embodiment of the present disclosure.

In FIGS. 5 and 6, "Inputs (4P)" represent CT tomography images generated by processing the four projection images taken by the CT scan unit 110, "AI results" are outputs of CT tomography images inferred by the AI model 121 of the CT scan apparatus 100 according to the embodiment of the present disclosure, and "C-A (360P)" are CT tomography images actually generated by using 360 images, that is, projection images taken at intervals of 1°.

As illustrated in FIGS. 5 and 6, it may be checked that CT tomography images output by the AI model 121 according to the embodiment of the present disclosure and CT tomography images generated by using actual 360 projection images are similar to each other.

That is, it is possible to generate a CT image with high picture quality and resolution by capturing only four projection images, so examination time may be decreased. As a result, it is possible to examine an electronic part by using CT images in real time in an in-line process.

In the embodiment of the present disclosure, as an example, the AI model 121 learns by using a CNN-based generative adversarial network (GAN).

In the above-described embodiment, the CT scan apparatus 100 according to the embodiment of the present disclosure has been illustrated to be applied to the examination of an electronic part. However, the technical idea of the present disclosure is not limited to the examination of an electronic part, and may be applied even to CT scan apparatuses for medical CT scan and dental CT scan.

Accordingly, it is possible to generate CT tomography images that provide higher picture quality and higher resolution with fewer shots even in medical CT scan including dentistry, so it may be expected to reduce examination time.

The embodiments of the present disclosure have been described with reference to the attached drawings, but those skilled in the art in the technical field to which the present disclosure belongs will understand that the present disclosure may be implemented in different specific forms without changing its technical idea or essential features. Therefore, the embodiments described above should be understood in all respects as illustrative and not restrictive.

### <Description of the Reference Numerals in the Drawings>

| | | | |
|---|---|---|---|
| 100: | CT scan apparatus | 110: | CT scan unit |

| | | | |
|---|---|---|---|
| 120: | CT image generation unit | 121: | AI model |
| 122: | Preprocessing module | 130: | Control unit |
| 140: | Learning DB | | |

### Industrial Applicability

It is possible to apply the present disclosure to the field of inspecting a printed circuit board (PCB) mounted as a main component of electrical and electronic products such as home appliances and computers, for example, a small electronic component such as a ball grid array (BGA) or a chip scale package (CSP).

## Claims

1. A CT scan apparatus comprising:
a CT scan unit configured to capture N projection images of an electronic part, and a CT image generation unit having an artificial intelligence-based AI model configured to receive the N projection images captured by the CT scan unit and output a CT tomography image of the electronic part.

2. The apparatus of claim 1, wherein the AI model is generated by learning N learning projection images, a first learning CT tomography image generated by the N learning projection images, and a second learning CT tomography image generated by M learning projection images (here, M > N) as learning data, wherein the AI model learns the second learning CT tomography image as output data.

3. The apparatus of claim 2, wherein the AI model learns the N learning projection images and the first learning CT tomography image as input data; and the CT image generation unit further comprises a preprocessing module configured to process the N projection images to generate a CT tomography image for input and input the CT tomography image to the AI model.

4. The apparatus of claim 2 or 3, wherein N is 4, and M is 8 or more.
